# EUROPEAN PATENT APPLICATION

(11) **EP 3 744 394 A1**
(43) Date of publication of application: **02.12.2020**
(21) Application number: 19176663.3
(22) Date of filing: 27.05.2019
(51) Int. Cl.: A61N 5/10

(54) **INTENSITY MODULATED PARTICLE THERAPY PLAN NORMALIZATION SCHEME**

(71) Applicant: Koninklijke Philips N.V., 5656 AG Eindhoven (NL)
(72) Inventor: RANGANATHAN, Vaitheeswaran, 5656 AE Eindhoven (NL); PERUMAL, Bojarajan, 5656 AE Eindhoven (NL); NATARAJAN, Ramar, 5656 AE Eindhoven (NL)
(74) Representative: de Haan, Poul Erik

(57) **Abstract**

A method and system for normalizing an IMPT plan are provided as well as an arrangement for planning IMPT and a computer program product comprising instructions to perform the method. The method comprises the steps of: receiving an IMPT plan for a subject to be treated, receiving anatomical image data of the subject comprising at least one ROI, and receiving at least one clinical goal associated with the ROI. The method further comprises identifying one or more deficiency areas of the IMPT plan within the ROI where the clinical goal is not met, and identifying the particle spots of the IMPT plan that are associated with the identified deficiency areas as critical particle spots. When the critical spots have been identified, the method comprises normalizing at least one of the deficiency areas by adjusting the intensity of the critical particle spots.

## Description

### FIELD OF THE INVENTION

The invention generally relates to planning intensity modulated particle therapy. In particular, the invention relates to normalizing an intensity modulated particle therapy plan. More specifically, the invention relates normalizing an intensity modulated particle therapy plan with localized plan deficiencies.

### BACKGROUND OF THE INVENTION

In intensity modulated particle therapy (IMPT) as well as conventional X-ray radiation therapy, target structures in a patient's body, such as tumours, are treated by subjecting them to irradiation. In IMPT tumours are treated by subjecting them to irradiation by a beam of particles, such as protons, carbon ions or helium ions. The treatment is delivered in such a way that the dose that is delivered to the target structures (TSs) is as high as possible, while at the same time the dose delivered to the surrounding healthy tissue and structures, usually referred to as organs at risk (OARs), is as low as possible. TSs and OARs are both regions of interest (ROIs) in the patient anatomy. A trade-off between the two types of ROI will have to be made. For this, clinical goals are prescribed. Clinical goals in planning the IMPT treatment, as well as X-ray radiation therapy treatment, specify the requirements for dose to be delivered to a TS. The clinical goals may also include requirements to constrain the dose to be delivered to each OAR. Clinical goals can be a minimum dose for the TS region, a maximum dose for an entire ROI region, a maximum dose for the hottest sub-region of a TS, a mean dose, or mean maximum or minimum dose for the a TS. Numerical optimization is typically used to determine the best total dose distribution to deliver. From this dose distribution the parameters for delivery are calculated that form the therapy plan.

Even after optimization, the dose distribution can still have deficiencies. For example, a maximum target dose for a TS may be violated or the specified volume coverage to the TS may not be met. These deficiencies can occur because a compromise needs to be made between the multiple clinical goals. In such a case, the clinician has several options available to get better coverage or reduce dose.

A fast approach to improve the dose delivered to a TS after optimization is normalization. In normalization the total intensity (or Motor Units, MU) of the therapy plan is increased or decreased. Current normalization approaches include directly tweaking the total MU of the plan.

With these normalization approaches, it is possible to improve the TS coverage or increase TS dose. However, these methods have significant disadvantages. By increasing the total intensity (MU) of the plan, or increasing the coverage volume to increase the overall dose to a TS volume, the normalized dose will also increase the maximum dose to the TS. The hot spots inside the target volume will become "hotter" in this process, which is not desirable. In addition, the OARs will also be proportionately overdosed.

The only currently available alternative to normalization is performing a full re-optimization with additional and/or modified clinical goals. Calculating this re-optimization is time consuming and the final result may still not be optimal. This is especially problematic for IMPT, because in these forms of therapy the dose computation and optimization cycle takes very long time to complete.

In "Pitfalls in normalization for intensity-modulated radiation therapy planning", Medical Dosimetry, Vol. 30, No. 4, pp 194-200, 2005, the authors recommend re-optimization over normalization.

### SUMMARY OF THE INVENTION

The current invention seeks to provide an alternative, faster way of improving dose coverage in an IMPT plan after optimization.

It is a further insight of the present invention that current normalization approaches in IMPT as well as the preference for re-optimization have been taken over from conventional X-ray radiation therapy planning methods without taking into account the particular problems of IMPT. The current invention additionally or alternatively seeks to provide an approach for normalizing an IMPT plan that takes into account the different nature of particle beams. Further advantages from the described invention will also be apparent to the skilled person.

Thereto a method and system for normalizing an IMPT plan are provided. Also, an arrangement for planning IMPT is provided. Further, a computer program product comprising instructions which, when executed, control a processor to perform the method for normalizing an IMPT plan is provided.

The method for normalizing an intensity modulated particle therapy plan comprises the steps of: receiving an intensity modulated particle therapy plan for a subject to be treated; receiving anatomical image data of the subject to be treated comprising at least one region of interest; and receiving at least one clinical goal associated with the at least one region of interest. With this data and information, the method further comprises identifying one or more deficiency areas of the intensity modulated particle therapy plan within the at least one region of interest where the at least one clinical goal is not met, and identifying the particle spots of the particle therapy plan that are associated with the identified deficiency areas as critical particle spots. When the critical spots have been identified, the method comprises normalizing at least one of the deficiency areas by adjusting the intensity of the critical particle spots. The method is preferably computer implemented.

IMPT plans define a pattern of locations where the particle beams are to be delivered, together with the intensity of the beam for each location. Because of the shape of a particle beam, and in particular the shape of the Bragg peak of the particle beam for particles such as protons, carbon ions and helium ions, a high dose is delivered to a relatively small volume. The location where the dose is delivered is referred to as a spot.

From the localized nature of the particle spots it follows that the individual particle beams that contribute to a specific part of the dose distribution can be identified. These individual beams deliver the spots associated to that area of the dose distribution. Contributions of these beams to other areas in the dose distribution are much smaller than the contribution to the associated dose area. With the indication of "dose area" in this context is meant a localized volume with in the patient anatomy that is part of a three dimensional dose distribution.

A "deficiency area" is a localized volume within an ROI of the patient anatomy where the clinical goals have not been met. The spots that deliver their particle dose to a deficiency area are critical particle spots. When the delivered particle dose in a sub-region of the ROI exceed the maximum dose that was prescribed, this sub-region is a deficiency area that is "hot". Hot deficiency areas can occur in a TS, but can also occur in an OAR. When the delivered particle dose in a sub-region of the ROI does not reach the minimum dose that was prescribed, this sub-region is a deficiency area that is "cold". This type of deficiency typically occurs in a TS. A cold deficiency area can result in a minimum prescribed dose not being reached or it can also have as a consequence that a prescribed percentage volume coverage of the TS is not reached.

According to one aspect, the method for normalizing an IMPT plan may comprise classifying the one or more deficiency areas as hot or cold deficiency areas.

The localized nature of the particles spots allows for identification of the critical spots that are associated with the deficiency area for hot as well as cold areas. The localized nature of the particle spots also allows for the spots to be identified that contribute to the dose that is delivered to an ROI. Not only critical particle spots can be identified in the ROI, but also the particle spots that deliver a dose that is conform the prescribed clinical goals. The regions within an ROI that receive a particle dose in accordance with the clinical goals are "normal" areas. The spots associated with these normal areas are normal particle spots.

According to a further aspect, the method for normalizing an IMPT plan may comprise identifying a normal areas of the intensity modulated particle therapy plan within the at least one ROI where the at least one clinical goals is met; and identifying the particle spots of the particle therapy plan that are associated with the identified normal area as normal particle spots. Identifying the normal areas and the associated normal particle spots has the advantage that more normalization strategies can be provided. Thereby the clinician has more options for fast improvement of the IMPT plans. For example, normalizing the at least one of the deficiency areas additionally or alternatively comprises adjusting the intensity of the normal particle spots.

Preferably, in the method the intensity, commonly expressed as MU, of the particle spots is adjusted as follows. The intensity of the particle spots is reduced when the deficiency area is a hot deficiency area, and the intensity of the particle spots is increased when the deficiency area is a cold deficiency area. The intensity can be adjusted for the critical hot or cold spots alone, or for a combination of the critical particle spots and the normal particle spots. In a known way of delivering particle therapy, the particle beam has as fixed energy level and can be switched on and off. The MU of the beam express the duration for which the beam is switched on. Intensity is increased or reduced by reducing by increasing or decreasing the MU, meaning leaving the beam on for a longer or shorter period of time.

According to a particular aspect of the method, normalizing the at least one of the deficiency areas comprises the steps of selecting a normalization goal and applying a normalization strategy corresponding to the normalization goal. The normalization goal defines the dose prescription for the ROI that should be achieved by the normalization. The dose prescription can, for example, be a maximum dose, a minimum dose, an average dose or a percentage volume coverage. The normalization goal may be entered into an input of the normalization unit by the clinician, but it may also be received automatically from a data file. A normalization strategy defines which group of particle spots will be adjusted and how. The group of particle spots can, for example be only critical particle spots, or a combination of critical and normal particle spots. The adjustment can be to increase or decrease the intensity to a predefined level of MU for a cold or hot deficiency area respectively. The normalization strategy may also include additional intensity constraints for particle spots in or adjacent to OARs for improved OAR sparing. For example, in OAR sparing, the particle spots that are in or adjacent to OARs are removed from the group of particle spots that will be adjusted.

Preferably, wherein the normalization goal corresponds to the at least one clinical goal associated with the at least one ROI. This has the advantage that no further data needs to be received by the system. An additional advantage is that the goals can be received automatically as part of the optimized IMPT plan.

Preferably, normalizing the at least one of the deficiency areas further comprises a step of determining whether or not the normalization goal has been achieved by applying the normalization strategy, and, if it is determined that the normalization goal has not been achieved, a step of applying an alternative normalization strategy corresponding to the normalization goal. Even more preferably, the steps of determining whether or not the normalization goal has been achieved and applying an alternative normalization strategy are repeated until it is determined that the normalization goal has been achieved. An advantage of including these steps as part of the method, is that the clinician can normalize the IMPT plan in a structured method and determine the appropriate normalization strategy. The method steps may also be computer-implemented. Computer implementation has the advantage that the method can be automated to normalize the plan automatically without the need for interaction with the clinician.

According to a further aspect of the method, multiple deficiency areas of the intensity modulated particle therapy plan are identified within the at least one ROI and each of the deficiency areas is normalized.

The system for normalizing an intensity modulated particle therapy plan comprises input configured to receive an intensity modulated particle therapy plan for a subject to be treated, an input configured to receive anatomical image data of the subject to be treated comprising at least one ROI, and an input configured to receive at least one clinical goal associated with the least one ROI. The system also comprises an identification module configured to identify one or more deficiency areas of the intensity modulated particle therapy plan within the at least one ROI where the at least one clinical goal is not met, and configured to identify the particle spots of the particle therapy plan that are associated with the identified deficiency areas as critical particle spots. The system further also comprises a normalization unit configured to normalize at least one of the deficiency areas by adjusting the intensity of the critical particle spots.

The system is preferably suitable for performing the above described method and its elements may be additionally configured to perform any of the method's embodiments.

Preferably, the normalization unit comprises a user interface. A user interface allows the clinician to be in control of the normalization process. Such an interface can allow the clinician to select which ROI and which deficiency region to normalize and which strategy to use. The clinician can also input the normalization goals. Alternatively, a user interface can allow the clinician to select automatic normalization. Thereby, the clinician is provided with a tool that he can use for IMPT plan normalization in accordance with his or her own knowledge and experience.

In a particularly advantageous embodiment, the user interface is part of a graphical user interface. This type of interface is user-friendly. A graphical user interface can also display an image of the dose distribution before normalization and after normalization to provide insight into the normalization results. The image can, for example, be a dose intensity map or a dose volume histogram.

The arrangement for planning IMPT comprises an imaging device configured to provide an image of a subject to be treated, a contouring tool configured to provide anatomical image data based on the image provided by the imaging device, an input for receiving clinical goals for the intensity modulated particle therapy, a unit configured to generate an intensity modulated particle therapy plan based on the anatomical image data and the clinical goals, and the system as described above for normalizing an IMPT plan.

An advantage of the current invention is that dose coverage in an IMPT plan after optimization can be improved faster than by using re-optimization.

Another advantage of the current invention is that a normalization approach for IMPT plans is provided with a reduced risk of creating and/or increasing hot spots in a TS and additionally
A further advantage of the current invention is that a normalization approach for IMPT plans is provided with a reduced risk of overdosing OARs.

Another further advantage of the current invention is that the quality of an IMPT plan can be improved with a lower increase of the overall intensity of the plan.

Further advantages of the described invention will also be apparent to the skilled person.

### BRIEF DESCRIPTION OF THE FIGURES

In the following drawings:
Fig. 1 schematically and exemplarily illustrates the particular shape of a Bragg-peak for a proton beam.
Fig. 2 schematically illustrates an example of an arrangement for planning IMPT that comprises a system for normalizing an IMPT plan.
Fig. 3 schematically and exemplarily illustrates a method for normalizing an IMPT plan.
Fig. 4a and 4b schematically and exemplarily illustrate normal areas and deficiency areas in for ROIs in an IMPT plan.
Fig. 5 schematically and exemplarily illustrates an alternative method for normalizing an IMPT plan.
Fig. 6 schematically and exemplarily illustrates a further alternative method for normalizing an IMPT plan.
Fig. 7 schematically illustrates an example of a user interface for a system for normalizing an IMPT plan.

### DETAILED DESCRIPTION OF THE INVENTION

In order to illustrate the principles of the present invention a method and system for normalizing an IMPT plan are described. Also, an arrangement for planning IMPT is described. The intensity modulated particle therapy is described with particular reference to therapy wherein protons are used as the particles. It is however to be appreciated that other particles, such as for example carbon ions or helium ions. may also be used instead of or in combination with protons.

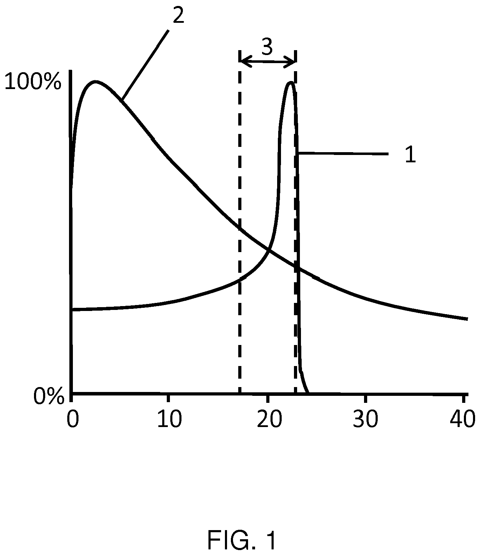

Figure 1 illustrates the shape of a Bragg-peak for a proton beam 1 used in IMPT. Beam intensity is shown as a function of penetration depth. For comparison, the intensity profile of a conventional X-ray beam 2 used for X-ray radiation therapy is also shown. In this figure, the vertical axis shows percentage of intensity and the horizontal axis shows penetration depth in cm. The target structure 3 is located between the dashed lines around 20 cm beneath the patient's skin. As can be seen in the figure, the proton beam 1 delivers a high intensity within the target structure 3 and a lower intensity at and directly beneath the skin. In particular when compared to the conventional X-ray beam 2. Figure 1 also shows the sharp fall-off of the intensity of the proton beam at the right-hand side of the target structure 3 providing the typical spot delivery of the radiation dose inside TS 3.

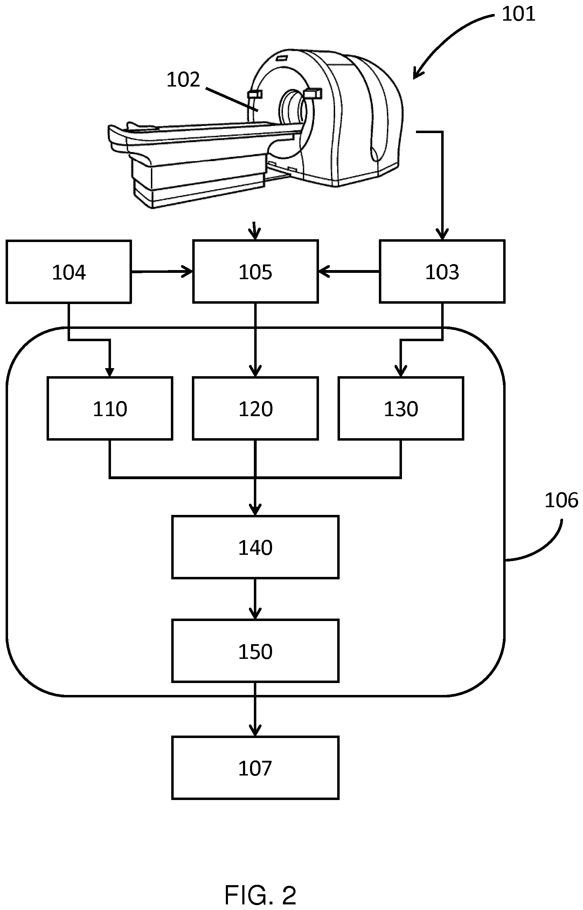

Figure 2 schematically illustrates an example of an arrangement 101 for planning IMPT that comprises a system for normalizing an IMPT plan 106. The arrangement 101 has an imaging device 102 that is configured to provide an image of a subject to be treated, a contouring tool 103 that is configured to provide anatomical image data based on the provided image, an input for receiving clinical goals 104 for the IMPT plan. The arrangement further comprises a unit configured to generate an IMPT 105 plan based on the anatomical image data and the clinical goals, and a system for normalizing the IMPT plan 106. The output of this arrangement is a normalized IMPT plan 107.

The imaging device 102 of the arrangement can be a structural imaging device, such as a CT, MR or ultrasound scanner, a functional imaging device, such as a PET or SPECT scanner or any combination thereof. It has particular advantages to use a combined PET-CT scanner, because a PET-CT image will allow the physician to identify OARs from the structural CT image as well as cancerous TSs from the PET functional image.

The image of the subject to be treated that is provided by the imaging device 102 is used as input for a contouring tool 103. The contouring tool delineates the OARs and TSs that are identified in the image. At least one TS will be delineated in the image. These delineations are the contours that form the anatomical image data for the subject to be treated. This delineation is commonly referred to as contouring. The contouring tool 103 may identify the contours automatically, or it may have an interface where the physician or a trained clinician inputs the contours manually. The contouring tool 103 may also use a combination of manual and automated contouring.

In order to generate the plan for the IMPT, clinical goals for the treatment are needed. The clinical goals comprise at least one goal for the at least one ROI, preferably a TS, but may comprise additional goals if there are multiple TSs and/or OARs. The arrangement provides an input 104 for these goals. The clinical goals can be determined by the physician based on the image provided by the imaging device 102, and may be based on clinical protocol. The clinical goals can be entered manually through a user interface, or, for example, uploaded automatically from a database using anatomical image data. Clinical goals will typically include maximum dose limits for the identified OARs and a minimum or average dose to be received by a TS. Clinical goals can also be biological dose limits such as Equivalent Uniform Dose (UED).

The unit configured to generate the IMPT plan 105 is a system that is configured to generate an initial, optimized IMPT plan. For generating this plan, a commercially available product such as Auto Plan may be used. Generating the IMPT plan is usually an interactive process performed by a physician.

Due to the highly localized nature of particle beams, such as proton, carbon ion or helium ion beams, IMPT is particularly sensitive to range and patient setup errors. Generating an IMPT plan therefore often involves and additional step of robust optimization wherein these errors are taken into account in order to calculate an IMPT plan with a reduced sensitivity to error. The choice of whether or not to apply robust optimization is made by the physician as part of the planning process. In robust optimization an overall plan objective value, which includes a clinical objective component and a patient setup error component, is minimized. Because in this optimization process compromises still need to be made between multiple objectives, robust optimized plans can still suffer from the same dose deficiencies as the original optimized plan. A maximum target dose for a TS may still be violated or the specified volume coverage to the TS may not be met. Robust optimized plans can therefore also benefit from the normalization approach according to the current invention.

The IMPT plan provided by unit 105, the clinical goals from input 104 and the anatomical image data provided by the contouring tool 103 are also used as input for the system for normalizing the IMPT plan 106. For this, the system 106 has and input 110 configured to receive at least one clinical goal, an input 120 configured to receive the IMPT plan for the subject to be treated, and an input 130 configured to receive the anatomical image data of the subject to be treated. This anatomical image data comprises at least one ROI, which is preferably a TS. These inputs may also be combined into two inputs or a single input. In the example of figure 2, an embodiment of the arrangement for planning IMPT is shown where the clinical goal input 110, the initial plan generator 105, and the contouring tool 103 are separate units to the system 106 for normalizing the IMPT plan. In such a setup, the inputs 110, 120 and 130 could be external data ports. In alternative embodiments, the plan generator 106, the contouring tool 103 and the system 106 could also be part of only two or even a single unit. In the example of a single unit, inputs 110, 120 and 130 would be part of an automated internal data transfer. In an alternative example, the input for receiving the clinical goals 110 can be combined with the input for clinical goals 103 and can be part of the unit configured to generate the IMPT 105.

The system further has an identification module 140 configured to identify one or more deficiency areas of the intensity modulated particle therapy plan within the at least one ROI where the at least one clinical goal is not met. Information on one or more ROIs is received as part of the anatomical image data, preferably in the form of ROI contours. The ROIs can be one or more TSs, and/or one or more TSs and one or more OARs. For each of the one or more ROIs, the identification module 140 determines whether or not the clinical goals are met for the area of the ROI. If the clinical goals are met throughout the ROI, there is no deficiency area. If the clinical goals are not met in one ore areas of the ROI, these areas are identified as deficiency areas. The identification module 140 is further configured to identify the particle spots of the particle therapy plan that are associated with the identified deficiency areas. The associated spots are identified as critical particle spots. In a preferred embodiment, the critical particle spots are grouped for each deficiency area. Such a group can be a simple list of the spots, or can be a separate ROI that is added to the anatomical image data.

The system further also has a normalization unit 150 configured to normalize at least one of the deficiency areas by adjusting the intensity of the critical particle spots. As part of the system for normalizing the IMPT plan 106, the normalization unit receives the clinical goals that provide information on the desired dosage in a deficiency area and the spots, preferably as groups, that have been identified as critical particle spots. The normalization unit adjusts the intensity of the critical particle spots to be at the level of the dosage that is desired according to the clinical goals. This can be done, for example, by scaling the MU of each critical spot with a scaling factor. Such a scaling factor may be determined by the ratio of desired dose level in the deficiency area to the average actual dose level in this area. This will yield a scaling factor higher than one for cold spots and a scaling factor of less than one for hot spots and is therefore suitable for both situations.

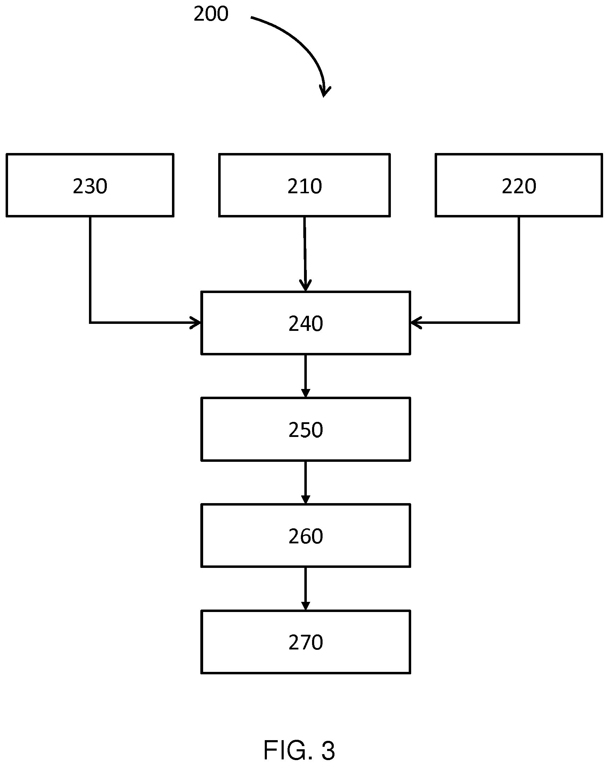

Figure 3 schematically and exemplarily illustrates a method for normalizing an IMPT plan 200. The method 200 comprises the steps of: receiving an intensity modulated particle therapy plan 210 for a subject to be treated; receiving anatomical image data 220 of the subject to be treated comprising at least one region of interest; and receiving at least one clinical goal 230 associated with the at least one region of interest. With this data and information, the method further comprises identifying one or more deficiency areas 240 of the IMPT plan within the at least one region of interest where the at least one clinical goal is not met, and identifying the particle spots of the particle therapy plan that are associated with the identified deficiency areas as critical particle spots 250. When the critical spots have been identified, the method comprises normalizing at least one of the deficiency areas by adjusting the intensity of the critical particle spots 260. The method may optionally comprise an additional step of providing the normalized control plan as output 270. This control plan can be used to operate the treatment device for delivery of the IMPT.

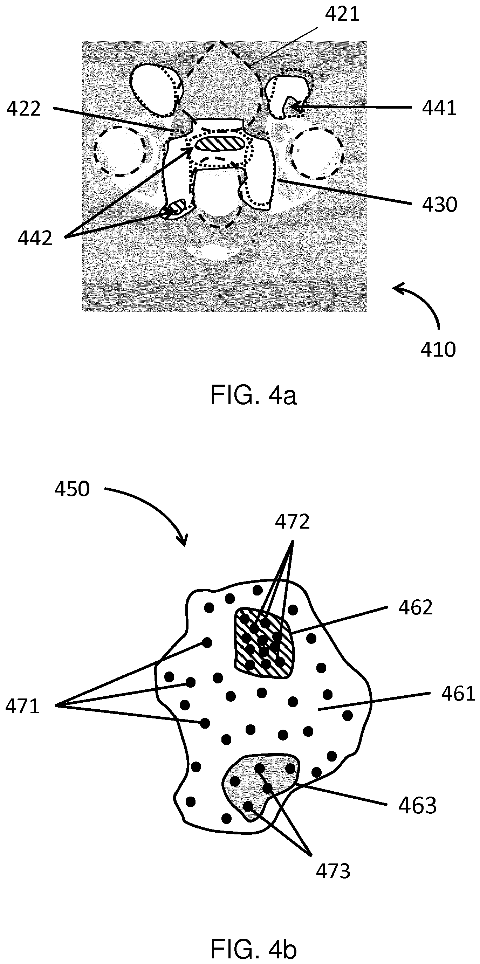

Figures 4a and 4b schematically and exemplarily illustrate normal areas and deficiency areas in for ROIs in an IMPT plan.

Figure 4a illustrates an IMPT plan 410 projected on a background of CT image of the patient. Anatomical image data is represented as dashed contours 421 and 422 with large dashed lines for OARs and small dotted lines for TSs. There are four OARs and five TSs in this example. Dosage level of the IMPT plan is indicated by solid isodose lines 430. Contours enclosing white shading represent a dosage level conforming to the clinical goal for the TSs. The white shaded are is a normal area. Contours enclosing hatched shading represent a dosage level that is higher than acceptable according to the clinical goals and an absence of shading represents a low dosage. This low dosage is lower than acceptable for a TS in accordance with the clinical goals, but preferred for the OARs. Figure 4a shows there is a cold deficiency area 441 in the upper right TS, hot deficiency area 442 for the lower left and center TSs.

Figure 4b schematically shows a TS 450 with the IMPT plan indicated in the form of locations of the particle spots. Dosage level has been indicated relative to the prescribed dose. The TS 450 has been delimited by a solid outer contour and the particle spots delivered by the particle beams of the IMPT plan are indicated as solid dots. The TS 450 of figure 4b has two deficiency areas 462 and 463 where the clinical goal for this TS 450 has not been met. There is a hot deficiency area 462 indicated with hatched shading, where the delivered dose is too high. There is also a cold deficiency area 463 indicated with grey shading, where the delivered dose is too low. In the remaining area of the TS the clinical goals are met ant this area forms the normal area 461.

Spots that lie in the deficiency areas are associated with these areas and are critical particle spots. Spots in hot deficiency area 462 are hot particle spots 472. Spots in cold deficiency area 463 are cold proton spots 473. The particle spots that lie in the normal area 461 are associated with that area and are normal proton spots 471.

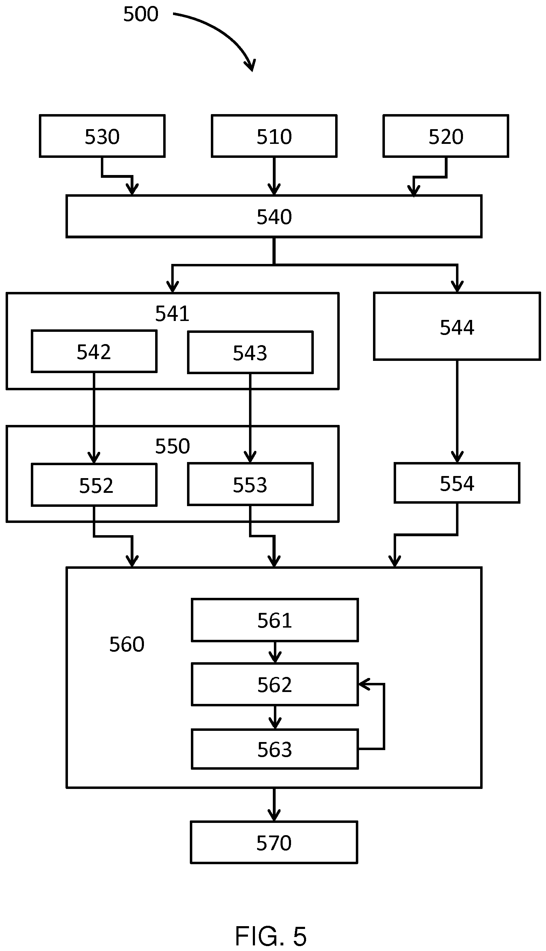

Fig. 5 schematically and exemplarily illustrates an alternative method for normalizing an IMPT plan 500. In this embodiment, the method 500 again comprises the steps of: receiving an intensity modulated particle therapy plan 510 for a subject to be treated; receiving anatomical image data 520 of the subject to be treated comprising at least one region of interest; and receiving at least one clinical goal 530 associated with the at least one region of interest. With this data and information, the method further comprises identifying one or more deficiency areas 540 of the IMPT plan within the at least one region of interest where the at least one clinical goal is not met.

After the deficiency areas have been identified, the method 500 comprises an additional step of classifying the one or more deficiency areas 541. The area is classified as a cold deficiency area 543 when the received dosage is too low to meet the prescribed clinical goal. Such a deficiency can occur in a TS and the clinical goal can, for example, be a prescribed minimum dosage or a percentage of target volume coverage. The deficiency area is classified as hot deficiency area 542 when the received dosage exceeds the prescribed clinical goal. Such a goal can be a maximum dosage for the ROI or a mean dosage prescribed for the ROI. Hot deficiency areas can occur in both TSs and OARs.

The method 500 has an optional additional step of identifying the one or more normal areas of the IMPT 544 for each of the one or more ROIs that have deficiency areas.

The method 500 further comprises identifying the particle spots of the IMPT plan associated with the one or more ROIs as critical particle spots 550. In this example, the particle spots associated with the deficiency areas that are classified as hot as identified as hot critical proton spots 552. The particle spots associated with the deficiency areas that are classified as cold are identified as cold critical proton spots 553. The method also further comprises identifying the particle spots of the IMPT plan that are associated with the identified one or more normal areas as normal particle spots 554.

The method 500 further comprises a step of normalizing at least one of the identified one or more deficiency areas 560. In the illustrated embodiment, all of the identified deficiency areas are normalized. This is preferably done sequentially for each deficiency area.

In the embodiment of Figure 5, normalizing a deficiency area comprises the steps of selecting a normalization goal 561 and applying a normalization strategy corresponding to the normalization goal 562. The normalization goal may be manually selected, but can also be determined automatically. Preferably, the selected goal corresponds to the prescribed clinical goal for the ROI.

The normalization strategy defines which group of particle spots will be adjusted and how. For example, when a deficiency area of a TS that has been identified and classified as cold, a normalization strategy can be to increase the intensity of the associated cold particle spots. Increasing the intensity will normally be done by increasing the MU of the beam. In another example, when a deficiency area of an ROI has been identified and classified as hot, a normalization strategy can be to decrease the intensity of the associated hot particle spots.

The normalization strategy may be pre-set based on the selected goal, or it can be manually or automatically selected from several options based on the selected goal. In the above example of a cold deficiency area in a TS, the normalization goal can be the clinical goal of a mean dose to be received by the TS. A pre-set strategy for this can be to increase the intensity level of the associated cold spots to be at the level of the prescribed mean dose.

Including the optional step of identifying the normal areas of the ROIs and the associated normal particle spots has the advantage that additional normalization strategies can be provided, thereby providing more options and better outcomes for fast improvement of the IMPT plans. For example, normalizing the at least one of the deficiency areas additionally or alternatively comprises adjusting the intensity of the ROI normal particle spots.

In the example of Figure 5, normalizing the deficiency area further comprises an optional step of determining whether or the normalization goal has been achieved 563 by applying a selected normalization strategy. If it is determined that the normalization goal has not been achieved, an alternative normalization strategy corresponding to the normalization goal is selected an applied. Preferably these steps 562, 563 are repeated until it is determined that the normalization goal has been achieved.

This method may optionally comprise an additional step of providing the normalized control plan as output 570. This control plan can be used to operate the treatment device for delivery of the IMPT.

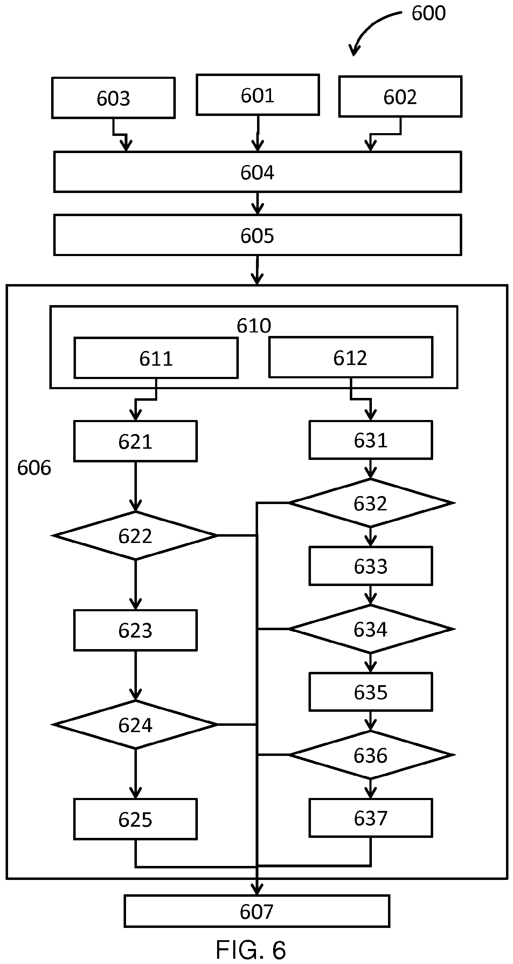

Figure 6 schematically and exemplarily illustrates a further alternative method for normalizing an IMPT plan. In this embodiment, the method 600 again comprises the steps of: receiving an intensity modulated particle therapy plan 601 for a subject to be treated; receiving anatomical image data 602 of the subject to be treated comprising at least one region of interest; and receiving at least one clinical goal 603 associated with the at least one region of interest.

With this data and information, the method further comprises identifying 604 one or more deficiency areas of the IMPT plan within the at least one region of interest where the at least one clinical goal is not met. This step 605 of the method also comprises identifying the one or more normal areas of the IMPT for each of the one or more ROIs that have deficiency areas. The method further comprises a step of identifying associated spots 605. In this step the particle spots associated with the identified one or more deficiency areas are identified as critical particle spots and the particle spots associated with the identified one or more normal areas are identified as normal particle spots.

The method 600 also comprises a step of normalizing at least one deficiency area 606. Method step 606 can be used to normalize a single deficiency area, and is described as such, but it may and applied sequentially to each area in case there are multiple deficiency areas. Method step 606 may also be advantageously applied for multiple deficiency area simultaneously in case there are multiple deficiency areas within the same ROI and with the same classification as hot or cold. In such a case, the multiple spots of the multiple deficiency area will be grouped after classification and then treated as a single deficiency area.

The method step of normalizing the deficiency area 606 is comprised of multiple further steps. First, the deficiency area is classified 610 as a hot 611 or cold 612 deficiency area. Depending on the classification, the method then a follows a series of steps for the purpose of achieving a pre-set normalization goal. The steps involve applying a normalization strategy 621, 623, 625, 631, 633, 635, 637, and checking if the normalization goal has been achieved 622, 624, 632, 634, 636. In of the steps 621, 623, 625, 631, 633, 635, 637, the normalization strategy is applied to the original IMPT plan. The normalization goal may correspond to the received clinical goal 603 for the ROI, but can also have been received separately from an additional user interface. If it is determined that the normalization goal has been achieved, the method step is completed. If the normalization goal has not been achieved, the method continues and applies the next normalization strategy.

For hot deficiency areas 611, first the deficiency area is normalized by reducing the intensity of only the hot critical spots 621. This is done by reducing the MU of the beams that deliver these spots. The MU can be reduced by multiplying them by a scaling factor of less than one, which can, for example by determined by the ratio of the prescribed maximum dose for the ROI to the average dose of the hot deficiency area. After this first normalization strategy is applied, it is determined whether or not the normalization goal has been reached 622. If this goal has been reached, normalization step 606 is completed.

If the goal has not been reached, normalization step 606 continues by applying an alternative normalization approach 623. In this alternative normalization approach 623 the deficiency area is normalized by reducing the intensity of the hot critical spots as well as the normal spots of the ROI. Again, this is done by reducing the MU of the beams that deliver these spots and the same scaling factor may be used for this. After this alternative normalization strategy is applied, it is determined whether or not the normalization goal has been reached 624. If this goal has been reached, normalization step 606 is completed. Due to the localized nature of the particle spots, it is most likely that the normalization goal will be reached by applying either of these normalization strategies 621, 623. However, if it is determined the goal has still not been reached, the normalization method step 606 is completed by the normalization of the IMPT plan in its entirety 625 by reducing the MU of all the beams of the plan.

For cold deficiency areas 612 in a TS, resulting in an insufficient volume coverage, first the deficiency area is normalized by increasing the intensity of only the cold critical spots 631. This is done by increasing the MU of the beams that deliver these spots. Also for cold spots, the MU can be changed by multiplying them by a scaling factor. In this case the scaling factor will be higher than one to provide for an increase. The scaling factor can, for example by determined by the ratio of the prescribed mean dose or the prescribed minimum dose for the ROI to the average dose of the cold deficiency area. After this first normalization strategy is applied, it is determined whether or not the normalization goal has been reached 632. If this goal has been reached, normalization step 606 is completed.

If the goal has not been reached, normalization step 606 continues by applying a first alternative normalization strategy 633. In this first alternative normalization approach 633, the deficiency area is normalized by increasing the intensity of the cold critical spots as well as the normal spots of the ROI. Again, this is done by increasing the MU of the beams that deliver these spots and the same scaling factor may be used for this. After this first alternative normalization strategy is applied, it is again determined whether or not the normalization goal has been reached 634. If this goal has been reached, normalization step 606 is completed.

If the goal has again not been reached, normalization step 606 continues by applying a second alternative normalization strategy 635. In this second alternative normalization approach 635, the deficiency area is normalized by increasing the intensity of only the normal spots of the ROI. This normalization strategy can be more successful in situations where the dose deficiency are in the TS is scattered over multiple small areas instead of one larger deficiency area. After this second alternative normalization strategy is applied, it is again determined whether or not the normalization goal has been reached 636. If this goal has been reached, normalization step 606 is completed.

Due to the localized nature of the particle spots, it is most likely that the normalization goal will be reached by applying one of these three normalization strategies 631, 633 or 635. However, if it is determined the goal has still not been reached, the normalization method step 606 is completed by the normalization of the IMPT plan in its entirety 637 by increasing the MU of all the beams of the plan.

As a last step, the normalizing method 600 comprise a step of providing the normalized control plan as output 607. This control plan can be used to operate the treatment device for delivery of the IMPT.

The normalization of the at least one deficiency in accordance with the above described method step 606 can be executed with user interaction, whereby the user reviews the result of the applied normalization strategy and then inputs whether or not the results is acceptable. This method also advantageously lends itself to fully automated execution, thereby providing a particularly fast method for normalizing the IMPT plan with improved quality.

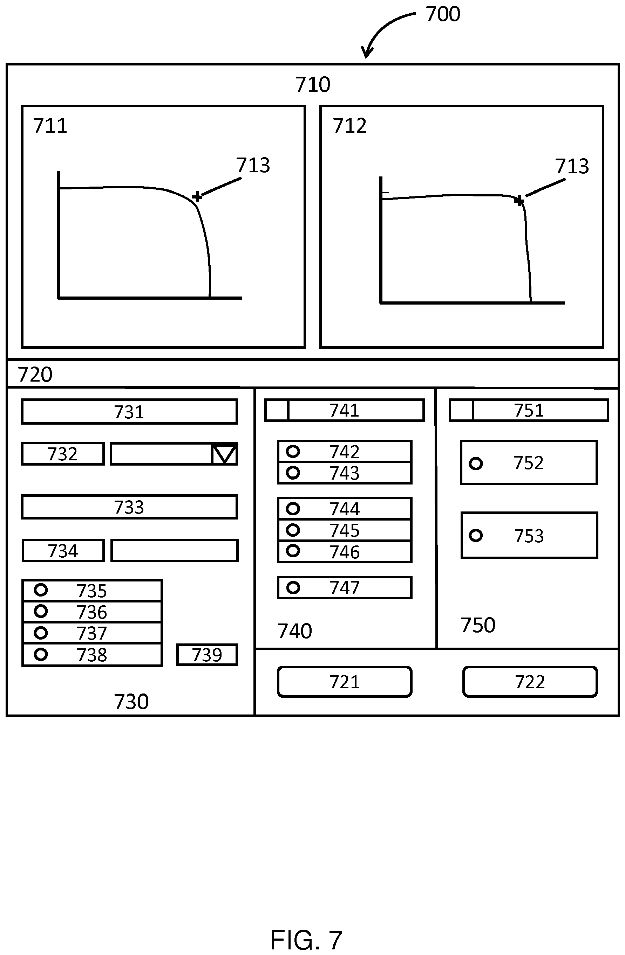

Figure 7 schematically illustrates an example of a user interface 700 for a system for normalizing an IMPT plan. Such a user interface can advantageously be part of a system for normalizing the IMPT plan and/or an arrangement for planning IMPT. Examples of such a system 106 and arrangement 101 are illustrated in Figure 2. The illustrated used interface can also advantageously be used to perform the methods illustrated in Figures 5 and 6.

The user interface comprises an IMPT plan normalization interface 720 configured for user interaction in the normalization process. The user of such an interface would typically be a clinician prescribing the treatment, or an IMPT planning technician. The exemplary user interface 700 illustrated in Figure 7 is a graphic user interface that includes an optional additional graphic representation 710 of dose information. Dose information on for the originally received IMPT plan 711 is displayed sided by side with dose information on the normalized IMPT plan 712. This allows the user to more easily compare both situations and determine if the applied normalization strategy has sufficiently reached the normalization goal.

The graphic representation can be a dose map, for example in false color to indicate dose levels and/or including iso-dose lines. An image of the patient anatomy in the form of a CT scan and/or anatomical data in the form of ROI contours can also be included in the graphic representation. Such a graphic representation would resemble the graphic shown in Figure 4a.

Figure 7 shows an alternative graphic representation of dose information in the form of a dose volume histogram (DVH) for a TS. Marker 713 represents the required dose level. As can be seen from the DVH of the original IMPT plan 711 in this example, the volume coverage of the TS is too low due to a cold deficiency area. After normalization of the cold deficiency area, DVH 712 shows the volume coverage of the TS has reached target level 713 without substantially increasing the maximum dose level in the TS.

IMPT plan normalization interface 720 comprises a target selection pane 730, a manual normalization pane 740, and an automatic normalization pane 750. The IMPT plan normalization interface 720 further comprises an execution button 721 and an accept button 722.

In target selection pane 730 the user can select the ROI comprising at least one deficiency area for which the dose level will be normalized. For this purpose, a target selection menu 731 is provided with ROI drop down menu 732. The drop down menu comprises a list of all the ROIs that comprise at least one of the identified deficiency areas. Target selection pane 730 further has normalization goal entry section 733. This section may be pre-filled with the received prescribed clinical goals, but also allows the user to separately enter different normalization goals. Normalization goal entry section 733 has a dose entry field 734 where the prescribed is entered, e.g. the prescribed dose in cGy. The entry section 733 further has a menu for indication the type of normalization goal. In this example, the menu is a list where a unique selection must be made. The list provides the options of the goal of ROI minimum dose 735, ROI mean dose 736, ROI maximum dose 377 and volume coverage percentage 378. The volume coverage option has an extra entry field 379 where the user enters the required percentage.

The used can choose between automatic and manual normalization by checking either the manual selection box 741 or the automatic selection box 751. When manual has been selected 741, the further menu of automatic pane 750 will be inactive, and when automatic has been selected 751, the further menu of manual pane 740 will be inactive.

In manual normalization mode, when manual selection box 741 has been checked, the user can select one of the listed options that is appropriate for the type of deficiency area. Preferably, a scenario may be followed corresponding to the method illustrated in Figure 6. For normalizing a hot deficiency area, the options of adjusting only hot critical particle spots 742 and of adjusting hot critical particle spots as well as normal particle spots 743 are provided. For normalizing a cold deficiency are, the options of adjusting only cold critical particle spots 744, of adjusting cold critical particle spots as well as normal particle spots 745, and of adjusting only normal particle spots 746 are provided. Preferably the options that are not applicable to the type of deficiency are not active and cannot be selected. This has the advantage of providing better focus to the user.

When manual normalization is selected by the user 741, and normalization is applied to a cold deficiency area in a TS, an additional option is provided and maybe selected for improved OAR sparing 747. When this OAR sparing option is used, the particle spots that are in or adjacent to OARs are removed from the group of particle spots that will be adjusted.

In automatic normalization mode, when automatic selection box has been checked, preferably the normalization method described with reference to Figure 6 is applied. This method distinguishes between hot and cold deficiency areas. When a selected ROI has both hot and cold deficiency area, the user needs to select which type should be normalized. For this purpose, a hot selection box 752 and a cold selection box 753 are provided. When the selected ROI has only one, or only one type of deficiency area, the system will automatically determine if this is a hot or cold area and normalize the deficiency are in the IMPT plan accordingly. In such a cases selection boxes 752 and 753 are pre-filled and function as indicator to the user.

After the target selection is complete and a normalization strategy has been selected, the execution button 721 can be used to normalize the initially received IMPT plan accordingly. In the example of Figure 7, the user can then evaluate the resulting normalized IMPT plan. If the result sufficiently meets the normalization goal, the used can accept the result and complete the normalization procedure by using accept button 721.

Any of the method steps disclosed herein, may be recorded in the form of a computer program comprising instructions which when executed on a processor cause the processor to carry out such method steps. The instructions may be stored on a computer program product. The computer program product may be provided by dedicated hardware as well as hardware capable of executing software in association with appropriate software. When provided by a processor, the functions can be provided by a single dedicated processor, by a single shared processor, or by a plurality of individual processors, some of which can be shared. Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system, or apparatus or device, or a propagation medium. Examples of a computer-readable medium include a semiconductor or solid state memory, magnetic tape, a removable computer diskette, a random access memory "RAM", a read-only memory "ROM", a rigid magnetic disk and an optical disk. Current examples of optical disks include compact disk - read only memory "CD-ROM", compact disk - read/write "CD-R/W", Blu-Ray™ and DVD. Examples of a propagation medium are the Internet or other wired or wireless telecommunication systems.

Variations to the disclosed embodiments can be understood and effected by those skilled in the art in practicing the claimed invention, from a study of the drawings, the disclosure, and the appended claims. It is noted that the various embodiments may be combined to achieve further advantageous effects.

In the claims, the word "comprising" does not exclude other elements or steps, and the indefinite article "a" or "an" does not exclude a plurality.

A single unit or device may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A method for normalizing an intensity modulated particle therapy plan, the method comprising:
receiving an intensity modulated particle therapy plan for a subject to be treated;
receiving anatomical image data of the subject to be treated comprising at least one region of interest;
receiving at least one clinical goal associated with the at least one region of interest;
identifying one or more deficiency areas of the intensity modulated particle therapy plan within the at least one region of interest where the at least one clinical goal is not met;
identifying the particle spots of the particle therapy plan that are associated with the identified deficiency areas as critical particle spots;
normalizing at least one of the deficiency areas by adjusting the intensity of the critical particle spots.

2. The method according to claim 1, further comprising:
classifying the one or more deficiency areas as hot or cold deficiency areas.

3. The method according to claim 1 or 2, further comprising:
identifying a normal area of the intensity modulated particle therapy plan within the at least one region of interest where the at least one clinical goals is met; and
identifying the particle spots of the particle therapy plan that are associated with the identified normal area as normal particle spots.

4. The method according to claim 3, wherein normalizing the at least one of the deficiency areas additionally or alternatively comprises adjusting the intensity of the normal particle spots.

5. The method according to any of claims 2-4, wherein the intensity of the particle spots is reduced when the deficiency area is a hot deficiency area; and the intensity of the particle spots is increased when the deficiency area is a cold deficiency area.

6. The method according to any of claims 1-5, wherein normalizing the at least one of the deficiency areas comprises the steps of selecting a normalization goal and applying a normalization strategy corresponding to the normalization goal.

7. The method according to claim 6, wherein the normalization goal corresponds to the at least one clinical goal associated with the at least one region of interest.

8. The method according to claims 6 or 7, wherein normalizing the at least one of the deficiency areas further comprises a step of determining whether or not the normalization goal has been achieved by applying the normalization strategy; and, if it is determined that the normalization goal has not been achieved, a step of applying an alternative normalization strategy corresponding to the normalization goal.

9. The method according to claim 8, wherein the steps of determining whether or not the normalization goal has been achieved and applying an alternative normalization strategy are repeated until it is determined that the normalization goal has been achieved.

10. The method according to any of claims 1-9, wherein multiple deficiency areas of the intensity modulated particle therapy plan are identified within the at least one region of interest and each of the deficiency areas is normalized.

11. A system for normalizing an intensity modulated particle therapy plan, the system comprising:
an input configured to receive an intensity modulated particle therapy plan for a subject to be treated;
an input configured to receive anatomical image data of the subject to be treated comprising at least one region of interest;
an input configured to receive at least one clinical goal associated with the least one region of interest;
an identification module configured to identify one or more deficiency areas of the intensity modulated particle therapy plan within the at least one region of interest where the at least one clinical goal is not met, and configured to identify the particle spots of the particle therapy plan that are associated with the identified deficiency areas as critical particle spots;
a normalization unit configured to normalize at least one of the deficiency areas by adjusting the intensity of the critical particle spots.

12. The system of claim 11, wherein the normalization unit comprises a user interface.

13. The system of claim 12, wherein the normalization unit user interface is part of a graphical user interface.

14. An arrangement for planning intensity modulated particle therapy comprising:
an imaging device configured to provide an image of a subject to be treated;
a contouring tool configured to provide anatomical image data based on the image provided by the imaging device;
an input for receiving clinical goals for the intensity modulated particle therapy;
a unit configured to generate an intensity modulated particle therapy plan based on the anatomical image data and the clinical goals; and
the system according to any of claims 11-13.

15. A computer program product comprising instructions which, when executed, cause a processor to perform the method according to of any one of the claims 1-10.
